(19) Europäisches Patentamt
European Patent Office
Office européen des brevets

(11) Publication number: **0 122 158 A2**

# (12) EUROPEAN PATENT APPLICATION

(21) Application number: **84302460.5**

(22) Date of filing: **11.04.84**

(51) Int. Cl.³: **C 07 D 501/20**, A 61 K 31/545

(30) Priority: **12.04.83 US 484125**

(43) Date of publication of application: **17.10.84**
**Bulletin 84/42**

(84) Designated Contracting States: **AT BE CH DE FR GB IT LI LU NL SE**

(71) Applicant: **ELI LILLY AND COMPANY, 307, East McCarty Street, Indianapolis Indiana 46285 (US)**

(72) Inventor: **Graves, Bernard James, Jr., 8324 Colonial Road, Indianapolis Indiana 46241 (US)**
Inventor: **Kukolja, Stjepan, 12123 Castle Overlook, Carmel Indiana 46032 (US)**

(74) Representative: **Crowther, Terence Roger et al, Erl Wood Manor, Windlesham Surrey GU20 6PH (GB)**

(54) **Improvements in or relating to benzothienyl cephalosporin antibiotics.**

(57) Novel 4, 5, 6, or 7-dihydro- and benzothienyl cephalosporins are useful in the oral treatment of gram positive bacterial infections.

EP 0 122 158 A2

# IMPROVEMENTS IN OR RELATING TO BENZOTHIENYL CEPHALOSPORIN ANTIBIOTICS

Among the clinically used cephalosporin antibiotics, few exhibit significant absorption from the gut and are therapeutically useful when administered orally. Cephalexin and cefaclor are two orally effective cephalosporin antibiotics which are highly absorbed. The majority of the cephalosporin antibiotics, however, are effective when administered parenterally. Considerable research work by chemists and microbiologists is directed toward the discovery and development of new orally effective semi-synthetic cephalosporin antibiotics with improved properties, for example, enhanced activity against staphylococci and streptococci, effectiveness against resistant microorganisms, and high absorptivity from the gut.

This invention relates to 7β-[2-amino-2-(benzothienyl)acetylamino]-3-substituted (or unsubstituted)-3-cephem-4-carboxylic acids, including the benzothien-4-yl, the benzothien-5-yl, benzothien-6-yl, and benzothien-7-yl isomers, and the salts and 2,3-dihydro derivatives thereof. The compounds are useful orally-effective antibiotics For example, 7β-[2-amino-2-(benzothien-4-yl)acetylamino]-3-methyl-3-cephem-4-carboxylic acid is effective against gram-positive and anaerobic bacteria when administered orally. The compounds are prepared by the N-acylation of a 7-amino-3-cephem nucleus compound with an amino-protected benzo- or dihydrobenzothienylglycine compound, or an activated derivative thereof.

The invention also provides intermediates useful in the preparation of the antibiotics and pharmaceutical formulations comprising the antibiotics.

In accordance with the invention, a compound of Formula (I):

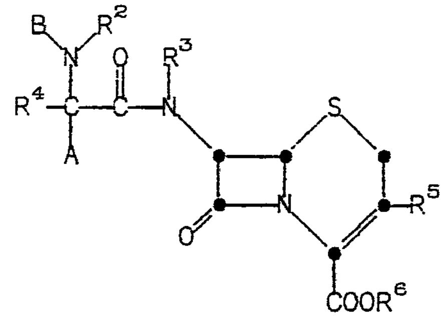

(I)

in which $R^4$ is

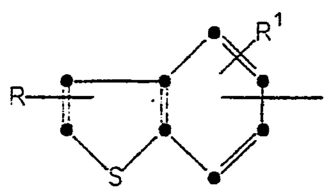

or

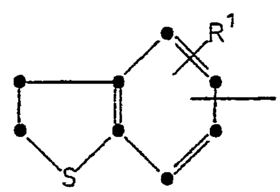

R is hydrogen, $C_1$-$C_3$ alkyl, halogen, nitro, amino, $C_1$-$C_4$ alkoxy, $C_1$-$C_4$ alkanoylamino, or $C_1$-$C_3$ alkylsulfonylamino;

$R^1$ is hydrogen, $C_1$-$C_3$ alkyl, halogen, hydroxy, or $C_1$-$C_4$ alkoxy;

A and B both are hydrogen, or taken together complete a double bond;

$R^2$ is hydrogen, an amino-protecting group, hydroxy, $C_1$-$C_4$ alkoxy or a carboxy-substituted alkoxy group or carboxy-substituted cycloalkoxy group of the formula

$$-\underset{d}{\overset{c}{\vdots}}\!\!\!C-(CH_2)_n-COOR^6$$

in which c and d, independently, are hydrogen or $C_1$-$C_3$ alkyl, or c and d taken together with the carbon to which they are bonded form a $C_3$-$C_6$ cycloalkyl ring; and n is 0 to 3;

$R^3$ is hydrogen, or $R^2$ and $R^3$ taken together are

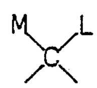

in which M and L, independently, are $C_1$-$C_4$ alkyl; provided that $R^2$ is hydroxy, $C_1$-$C_4$ alkoxy, carboxy-substituted alkoxy or carboxy-substituted cycloalkoxy only when A and B complete a double bond, and that A and B, both are hydrogen when $R^3$ is other than hydrogen;

$R^5$ is hydrogen, methyl, halogen, methoxy, vinyl, or a substituted methyl group represented by the formula

$$-CH_2-R^{5'}$$

in which $R^{5'}$ is methoxy, acetoxy, or carbamoyloxy;

$R^6$ is hydrogen, or a carboxy-protecting group; or a pharmaceutically-acceptable salt thereof, is useful as an orally-active antibiotic or an intermediate thereto.

In another aspect of the invention, there is provided a process for preparing a compound of Formula (I), or a pharmaceutically-acceptable salt thereof which comprises

(A) acylating a compound of Formula (II):

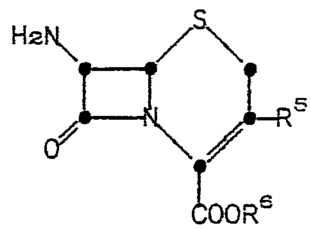

(II)

with an acylating agent of Formula (III):

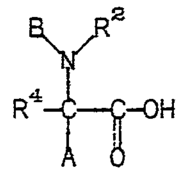

(III),

or an activated derivative thereof, in which A, B, $R^2$, $R^4$, $R^5$ and $R^6$ are as defined previously, followed, optionally, by removal of any amino- or carboxyl-protecting groups present;

(B) deblocking of a protected acid of Formula (I) in which $R^6$ is a carboxy-protecting group to provide a compound of Formula (I) in which $R^6$ is hydrogen;

(C) removal of an amino-protecting group, $R^2$, from a compound of Formula (I) to provide a compound of Formula (I) in which $R^2$ is hydrogen;

(D) when it is desired to form a compound in which $R^2$ and $R^3$, when taken together form a group of the Formula:

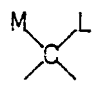 ,

reacting a compound of Formula (I), in which $R^2$ and $R^3$ both are hydrogen, with a ketone of the Formula:

M-C(O)-L

in which M and L are as defined previously or,

(E) reducing a compound of Formula (I) in which A and B are taken together to form a double bond and $R^2$ is hydroxy or methoxy, to produce a compound of Formula (I) in which A, B, and $R^2$ are hydrogen; and,

(F) if desired, salifying a compound of formula (I),

(G) or, if desired, converting a salt of a compound of Formula (I) to the free amine or acid.

In the above formula "halogen" refers to fluoro, chloro, or bromo; "$C_1$-$C_3$ alkyl" refers to methyl, ethyl, n-propyl, and isopropyl; and $C_1$-$C_4$ alkoxy refers to methoxy, ethoxy, n-propoxy, iso-propoxy, n-butoxy, iso-butoxy, and t-butoxy.

The compounds of Formula (I), having an asymmetric carbon atom, can exist as the D,L- mixture or as the individual D- or L-isomers. The compounds also can exist in the internal salt form or zwitterionic form such as that represented by the Formula:

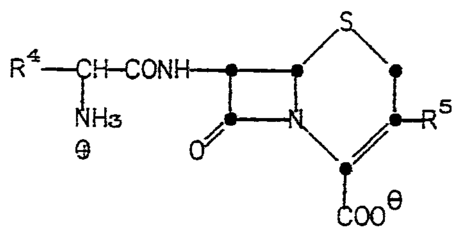

As shown in Formula (I), the compounds of the invention may be characterized by the nature of the bonding in the glycyl side chain. The glycyl moiety $-CH(NH_2)-CO-$ is bonded to a carbon of the phenyl ring of the benzothienyl moiety rather than to a carbon of the thienyl or the dihydrothienyl ring.

Pharmaceutically-acceptable salts of the Formula (I) compounds may include the acid addition salts of the amino group of the side chain ($R^2$ is hydrogen) such as the hydrochloride and hydrobromide salts, as well as the salts formed with the carboxy group, when $R^6$ is hydrogen, such as the alkali

metal and alkaline earth metal salts e.g. sodium, potassium, and calcium salts. Likewise, the ammonium and substituted ammonium salts can be formed, for example, the benzylammonium, dibenzylammonium, 2-hydroxyethylammonium, di-(2-hydroxyethyl)ammonium, cyclohexylammonium, dicyclohexylammonium, methylammonium, diethylammonium, dipropylammonium and like ammonium salts formed with ammonia and primary and secondary organic amines. Other amine salts may also be used such as those formed with procaine and abietylamine. "Pharmaceutically-acceptable salts" are those salts useful in the chemotherapy of warm-blooded animals.

Examples of compounds of the invention represented by Formula (I) in which $R^4$ is benzothienyl are:

7β-[2-amino-2-(benzothien-4-yl)acetylamino]-3-methyl-3-cephem-4-carboxylic acid,

7β-[2-amino-2-(benzothien-5-yl)acetylamino]-3-methyl-3-cephem-4-carboxylic acid,

7β-[2-amino-2-(benzothien-4-yl)acetylamino]-3-chloro-3-cephem-4-carboxylic acid,

7β-[2-amino-2-(benzothien-6-yl)acetylamino]-3-methoxy-3-cephem-4-carboxylic acid,

7β-[2-amino-2-(benzothien-5-yl)acetylamino]-3-methoxymethyl-3-cephem-4-carboxylic acid,

7β-[2-amino-2-(benzothien-7-yl)acetylamino]-3-methyl-3-cephem-4-carboxylic acid,

7β-[2-amino-2-(benzothien-4-yl)acetylamino]-3-acetoxymethyl-3-cephem-4-carboxylic acid,

7β-[2-amino-2-(7-hydroxybenzothien-4-yl)-acetylamino]-3-methyl-3-cephem-4-carboxylic acid,

7β-[2-amino-2-(6-hydroxybenzothien-4-yl)-acetylamino]-3-methyl-3-cephem-4-carboxylic acid,

7β-[2-amino-2-(4-hydroxybenzothien-7-yl)-acetylamino]-3-chloro-3-cephem-4-carboxylic acid,

7β-[2-amino-2-(5-chlorobenzothien-4-yl)-acetylamino]-3-methoxy-3-cephem-4-carboxylic acid,

7β-[2-amino-2-(6-methylbenzothien-7-yl)-acetylamino]-3-bromo-3-cephem-4-carboxylic acid,

7β-[2-amino-2-(7-methoxybenzothien-4-yl)-acetylamino]-3-methyl-3-cephem-4-carboxylic acid,

7β-[2-amino-2-(7-methoxybenzothien-4-yl)-acetylamino]-3-acetoxymethyl-3-cephem-4-carboxylic acid,

7β-[2-amino-2-(benzothien-6-yl)acetylamino]-3-methyl-3-cephem-4-carboxylic acid,

7β-[2-amino-2-(benzothien-4-yl)acetylamino]-3-cephem-4-carboxylic acid,

7β-[2-amino-2-(2-aminobenzothien-4-yl)acetyl-amino]-3-methyl-3-cephem-4-carboxylic acid,

7β-[2-amino-2-(2-acetylaminobenzothien-7-yl)acetylamino]-3-methoxy-cephem-4-carboxylic acid,

7β-[2-amino-2-(3-methylsulfonylaminobenzo-thien-5-yl)acetylamino]-3-methyl-3-cephem-4-carboxylic acid, and

7β-[2-amino-2-(benzothien-4-yl)acetylamino]-3-carbamoyloxymthyl-3-cephem-4-carboxylic acid; or a pharmaceutically-acceptable salt thereof.

Examples of dihydrobenzothienyl cephalosporins provided by the invention in which $R^4$ of Formula (I) is dihydrobenzothienyl are represented by the following Formula:

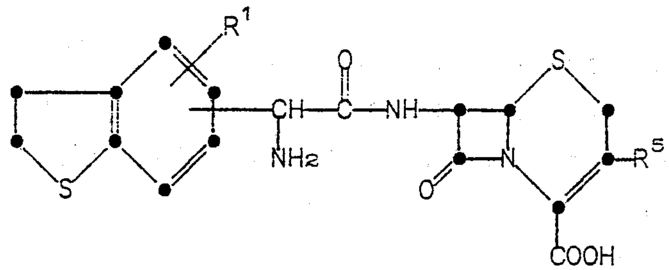

| $R^1$ | $R^5$ | isomer |
|---|---|---|
| H | $CH_3$ | 4-yl |
| H | $CH_3$ | 6-yl |
| $CH_3$ | H | 4-yl |
| H | Cl | 4-yl |
| H | $OCH_3$ | 5-yl |
| Cl | acetoxymethyl | 4-yl |
| $CH_3O$ | carbamoyloxymethyl | 5-yl |
| Cl | $CH_3$ | 4-yl |
| Br | $CH_3$ | 7-yl |
| H | $CH_3OCH_2$ | 5-yl |

A preferred group of compounds of Formula (I) are those in which $R^4$ is benzothienyl and R and $R^1$ are hydrogen and $R^5$ is methyl, methoxy, or chloro. Another preferred group is represented when R is hydrogen, methyl, or chloro, $R^1$ is hydrogen, hydroxy, methoxy or ethoxy.

The compounds provided by the invention can be prepared by various routes. One route comprises the N-acylation of a 7-amino nucleus compound represented by Formula (II):

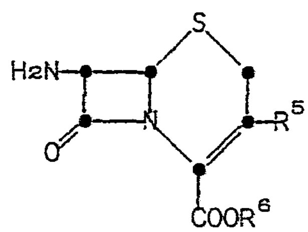

(II)

in which $R^5$ has the same meaning as defined above and $R^6$ is a carboxy protecting group, with the amino-protected benzothienylglycyl or dihydobenzothienylglycyl compound, or an activated derivative thereof, represented by Formula (III):

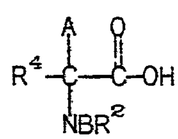

(III)

in which A, B, $R^4$, and $R^2$ are as defined earlier. In such reactions, $R^2$ normally represents an amino-protecting group, as defined later.

The amino-protected and esterified acylation products represented by the Formula:

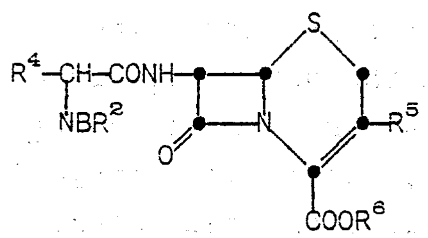

may be deprotected or deesterified to produce the corresponding compound of Formula (I).

Examples of carboxy-protecting groups $R^6$ are the conventional protecting groups for cephalosporins such as, for example, t-butyl, 2-iodoethyl, methoxymethyl, allyl, 2,2,2-trihaloethyl, benzyl, substituted benzyl such as p-methoxybenzyl, p-nitrobenzyl, p-methylbenzyl, and diphenylmethyl, trialkylsilyl such as trimethylsilyl, and like protecting groups. Other useful carboxy-protecting groups will be recognized by those skilled in the art. A discussion of such protecting groups may be found in "Protective Groups in Organic Chemistry", J.F.McOmie, Ed., Plenum Press, New York, N.Y., (1973) and "Protective Groups in Organic Synthesis", Greene, Ed., John Wiley and Sons, N.Y. (1981), both of which are incorporated herein by reference.

The term "activated derivative" means a derivative which renders the carboxyl function of the acylating agent reactive to coupling with a primary amino group to form the amide bond which links the acyl side chain to the nucleus. Suitable activated derivatives, their methods of preparation, and their use as acylating agents for a primary amine will be recognized by those skilled in the art. Preferred activated derivatives are: (a) an acid halide (e.g. an acid chloride or bromide) (b) an acid anhydride, (c) the carboxazide or (d) an activated ester. Other methods for activating the carboxyl function include reaction of the carboxylic acid with a carbodiimide (e.g. N,N'-dicyclohexylcarbodiimide or N,N'-diisopropylcarbodiimide) to give a reactive intermediate which is reacted *in situ* with the nucleus of Formula (III). This reaction is discussed in more detail later.

Examples of active esters are those formed with N-hydroxy heterocyclics such as hydroxybenzotriazole, hydroxytriazole, N-hydroxysuccinimide and the like. Anhydrides of the benzothienylglycine useful in the N-acylation are those formed with the chloroformates such as methyl chloroformate, ethyl chloroformate or isobutyl chloroformate.

During the acylation, the amino group of the benzothienylglycine or the dihydro-derivative desirably is protected to prevent its acylation in competition with the 7-amino nucleus. Useful amino-protecting groups are those commonly employed for the temporary protection of amino groups in the β-lactam antibiotics art. Examples of protecting reagents which

may be used include, for example, the active methylene compounds such as methyl acetoacetate and ethyl acetoacetate which form enamines with the amine; the alkoxy-carbonyl halides, substituted alkoxycarbonyl halides and alkenyloxycarbonyl halides such as the $C_1$-$C_4$ alkoxy-carbonyl chlorides e.g. ethoxycarbonyl chloride, t-butoxycarbonyl chloride, trichloroethoxycarbonyl chloride, benzyloxycarbonyl chloride, p-nitrobenzyloxycarbonyl chloride and allyloxycarbonyl chloride; bicyclic carbonyl chlorides such as adamantyloxycarbonyl chloride; and cycloaliphatic carbonyl chlorides such as cyclopentyloxycarbonyl chloride and cyclohexylcarbonyl chloride. Other amino-protecting groups will be recognized by those skilled in the art. Such protecting groups are discussed in "Protecting Groups in Organic Chemistry", and "Protective Groups in Organic Synthesis", supra.

Those skilled in the art will appreciate that when R in Formula (I) is an amino group, it also desirably is protected during the acylation to prevent unwanted N-acylation at this amino group. Any of the above-mentioned amino-protecting groups can serve for the temporary protection of compounds in which R is amino.

The acylation can be carried out with the amino group protected as the salt e.g. the hydrochloride salt. For example, the benzothienylglycyl chloride hydrochloride is used to acylate the 7-amino nucleus protected as a silyl ester e.g. the trimethylsilyl ester. After the acylation is complete, the silyl ester is hydrolyzed and the product obtained as the free

amine, free acid at its isoelectric point. This acylation method is illustrated by the following reaction scheme in which 7-ADCA trimethylsilyl ester is acylated with 2-amino-2-(benzothien-4-yl)acetyl chloride hydrochloride.

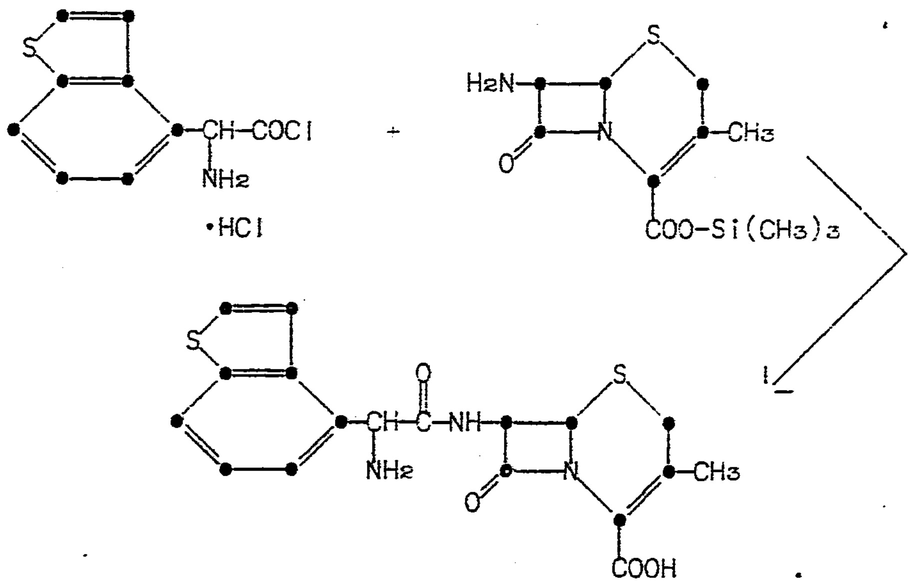

The amino-protected benzothienylglycine or the dihydro derivative, as the free acid, can be used to acylate the 7-amino nucleus by coupling the acid and the amine with a dehydrating/coupling reagent. Dehydrating/ coupling reagents which may be used are, for example, the carbodiimides such as dicyclohexylcarbodiimide and dibutylcarbodiimide; and the common coupling reagent EEDQ (1-ethoxycarbenyl-2-ethoxy-1,2-dihydroquinoline). For example, 2-t-butyloxycarbamido-2-(benzothien-5-yl)acetic acid is reacted in a dry inert organic solvent such as acetonitrile with p-nitrobenzyl 7-amino-3-chloro-3-cephem-4-carboxylate in the presence of excess EEDQ to provide p-nitrobenzyl 7β-[2-(t-butyloxycarbonylamino)-2-(benzothien-5-yl)acetylamino]-3-chloro-3-cephem-4-carboxylate. The product may then be reduced with zinc and acetic acid to remove the p-nitrobenzyl ester group and the amino-protecting group may be removed by treatment with trifluoroacetic acid.

The 7-amino nucleus compounds of Formula (II) used in the acylation are known and can be prepared by well-known procedures. 7-ADCA ($R^5 = CH_3$) is described by Stedman, J. Med. Chem., 7, 117 (1964) and by Chauvette et al., J. Org. Chem., 36, 1259 (1971); the 3-halo nucleus and the 3-methoxy nucleus ($R^5$ = halo or $OCH_3$) are described by Chauvette in U.S. Patent Nos. 4,064,343 and 3,917,587 respectively; and the 3-vinyl nucleus ($R^5 = -CH=CH_2$) is described in U.S. Patent No. 3,994,884.

The compounds of Formula (I) in which $R^5$ is methyl also can be prepared by the N-acylation of 7-amino-3-exomethylenecepham-4-carboxylic acid esters (U.S. Patent No. 3,932,393) followed by isomerization of the 3-exomethylenecepham to the 3-cephem (exo to endo). The N-acylation of the 3-exomethylenecepham nucleus is carried out by any of the N-acylation methods described earlier. For example, 2-(t-butyloxycarbonylamino)-2-(benzothien-6-yl)acetic acid is converted to the anhydride formed with methyl chloroformate in the presence of triethylamine, and the anhydride then is reacted with p-nitrobenzyl 7-amino-3-exomethylenecepham-4-carboxylate as shown below:

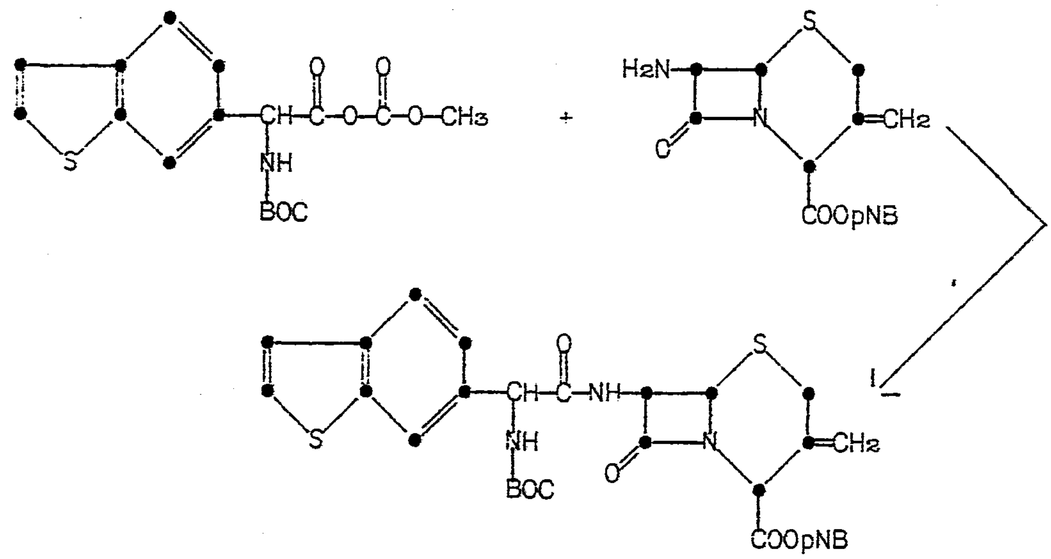

in which BOC is t-butyloxycarbonyl and pNB is p-nitrobenzyl. The product is then treated with a mixture of a highly polar aprotic solvent such as dimethylacetamide and a tertiary amine such as triethylamine to provide, via isomerization of the 3-exo double bond, the corresponding 3-methyl-3-cephem ester. The pNB ester is removed by reduction with zinc and acetic acid and the BOC group is removed with trifluoroacetic acid.

The compounds of Formula (I) in which $R^5$ is halo or methoxy also can be obtained by an alternative process. This alternative process employs a 7-amino-3-hydroxy-3-cephem-4-carboxylic acid ester represented by the following Formula:

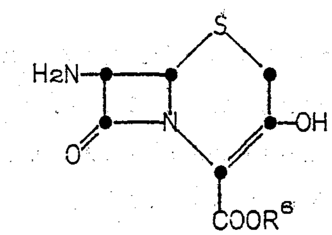

in which $R^6$ has the same meanings as defined earlier. The acylation of the 3-hydroxy nucleus ester with an amino-protected benzothienylglycine or dihydro derivative thereof is carried out in the presence of water to prevent the competitive 3-hydroxy acylation which can occur under anhydrous conditions (U.S. Patent No. 3,917,587). Following acylation, the 3-hydroxy group is converted to the 3-methoxy group with diazomethane or to the 3-halo compound as described by U.S. Patent No. 4,064,343. After the methylation or halogenation, the ester group and the amino-protecting group are removed to provide the desired 3-halo or 3-methoxy compound.

In yet a further method for preparing compounds of Formula (I) in which $R^5$ is methyl, methoxy, or chloro, a 7-aminocephalosporanic acid ester is acylated with the desired benzothienylglycine or dihydrobenzothienyl glycine derivative and the product is reacted with a thiol such as a $C_1$-$C_4$ alkyl mercaptan or with thiourea to form, the 3-alkylthio or isothiouronium derivative, respectively, by nucleophilic displacement of the 3-acetoxy group. The thio derivative is then subjected to reductive displacement with Raney nickel and hydrogen or with zinc and formic acid in DMF to provide the 3-exomethylenecepham compound. The latter is then isomerized to the 3-methyl-3-cephem as described above. The 3-exomethylenecepham can be reacted with ozone in the cold to provide the 3-hydroxy-3-cephem and the latter converted to the 3-halo or 3-methoxy compound as described earlier. The conversion of the 3-acetoxymethyl compound to the 3-exomethylenecepham is performed according to the methods described by Chauvette in U.S. Patent No. 3,932,393.

The compounds represented by Formula (I) also can be obtained by yet a further general preparative method. According to this method, a 2-(benzothienyl)-2-oximino (or alkoximino)acetic acid (A and B form a double bond) or the dihydro derivative represented by the Formula:

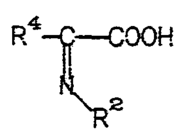

,

or an activated derivative thereof, is used to N-acylate a 7-amino nucleus compound

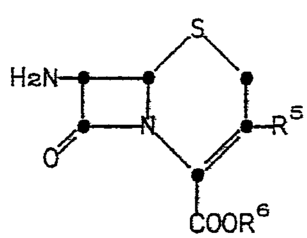

to provide a 7β-[2-(benzothienyl or dihydrobenzothienyl)-2-oximino (or alkoxyimino)acetylamino]-3H or (3-substituted)-3-cephem-4-carboxylic acid ester represented by Formula (IV).

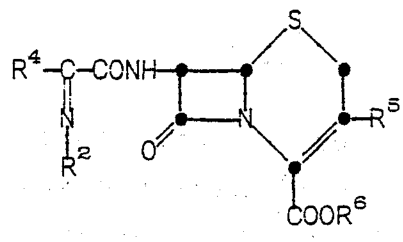

(IV)

In this formula, $R^4$, $R^5$ and $R^6$ have the same meanings as defined earlier and $R^2$ is hydrogen or $C_1$-$C_4$ alkyl. The acylation is carried out by the methods described previously for the N-acylation of a 7-amino nucleus compound with an amino-protected benzothienylglycine. The oximino group is then reduced to the amino group and the ester group may be removed to provide the corresponding compound of Formula (I). The reduction may be carried out chemically or by catalytic hydrogenation. Chemical reduction is preferred, for example, with zinc and an acid such as formic acid or acetic acid.

According to a further aspect of this invention, the oximino esters represented by Formula (IV), the free acids obtained by deesterification of the esters, and certain derivatives thereof are provided. The oximino compounds provided are represented by Formula (V):

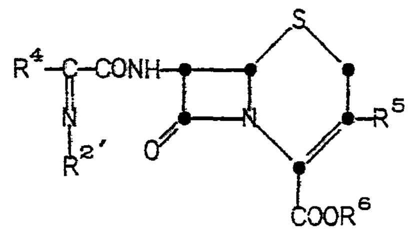

(V)

Compounds included within this formula are those in which $R^4$, $R^5$ and $R^6$ have the same meanings as defined for Formula (I); $R^{2'}$ is hydroxy, $C_1$-$C_4$ alkoxy, or a carboxy-substituted alkoxy or carboxy-substituted cycloalkoxy group of the Formula:

$$-\overset{c}{\underset{d}{C}}-(CH_2)_n-COOR^6$$

in which $R^6$ has the same meanings as above, c and d independently, are hydrogen or $C_1$-$C_3$ alkyl or c and d taken together with the carbon to which they are bonded form a $C_3$-$C_6$ cycloalkyl ring, and n is 0 to 3; or a pharmaceutically-acceptable salt thereof.

The oximino compounds of Formula (V) in which $R^{2'}$ is hydrogen or $C_1$-$C_4$ alkoxy, and $R^6$ is a carboxy-protecting group are useful intermediates in the preparation of compounds of Formula (I). The compounds in which $R^6$ is hydrogen or a pharmaceutically-acceptable salt thereof, possess antibacterial activity and are useful in controlling the growth of microorganisms

pathogenic to man and animals. The latter compounds possess inhibitory activity against a broader spectrum of microorganisms than do the Formula (I) compounds. The oximino free acids of Formula (V) or a salt form thereof, possess activity against gram-negative and gram-positive bacteria such as staphylococcus, streptococcus, salmonella, proteus, E. coli, and klebsiella.

The compounds represented by the Formula (V) in which $R^{2'}$ is a carboxy-substituted alkoxy or cycloalkoxy group are prepared with the compounds in which $R^{2'}$ is hydroxy. The free hydroxime is alkylated with a carboxy-substituted alkyl halide represented by the Formula:

$$X-\overset{c}{\underset{d}{C}}-(CH_2)_n-COOR^6$$

in which X is chloro or bromo, $R^6$ is a carboxy-pro-tecting group as defined above, and c, d, and n are as defined earlier. The alkylation is carried out under anhydrous conditions in an inert organic solvent such as dimethylformamide, dimethylacetamide, tetrahydrofuran, or acetonitrile in the presence of a base e.g. sodium hydride. Examples of such carboxy-substituted alkoxy groups are carboxymethoxy, 2-carboxyethoxy, 2-carboxy-prop-2-oxy, 3-carboxypropoxy, 3-carboxybut-3-oxy and 2-(carboxymethyl)prop-2-oxy. Preferably, the free oxime of the α-oximino-acetic acid benzothienyl side chain is alkylated with the carboxy-substituted alkyl halide and the derivative obtained is used to acylate the desired 7-amino nucleus compound by one of the acylation

methods described earlier. Examples of carboxy-substituted cycloalkoxy groups are 1-carboxycyclobut-1-yloxy, 1-carboxymethylcyclobut-1-yloxy, 1-carboxycyclopent-1-yloxy, and 1-carboxycyclohex-1-yloxy.

Preferred compounds of the invention are represented by Formula (V) when R and $R^1$ are hydrogen, $R^{2'}$ is methoxy or 2-carboxyprop-2-yloxy, $R^5$ is methyl, chloro, or methoxy and the oximino group is in the syn orientation. Other preferred compounds are represented when R is methyl, and $R^1$ is hydrogen, hydroxy, or chloro. An example of a preferred compound is 7β-[2-(benzothien-4-yl)-2-syn-methoxyiminoacetylamino]-3-methyl-3-cephem-4-carboxylic acid.

The dihydro- and benzothienylglycine derivatives employed in the acylation of the 7-amino nucleus compounds are obtained with a benzothienylglyoxylic acid ester represented by the Formula:

$$R^4-\underset{\underset{O}{\|}}{C}-COOR^7$$

in which $R^7$ is $C_1$-$C_4$ alkyl and $R^4$ has the same meanings as defined for Formula (I). The glyoxylic ester derivative is converted to an oxime with hydroxylamine or to an O-substituted oxime with an O-substituted hydroxylamine, for example, methoxylamine. The oxime or O-oxime is then reduced by catalytic hydrogenation over palladium on carbon catalyst, or preferably with zinc dust and formic acid to the glycine ester, and the glycine ester is saponified to the glycine. This reaction sequence is illustrated with a benzothienylglycine in the following:

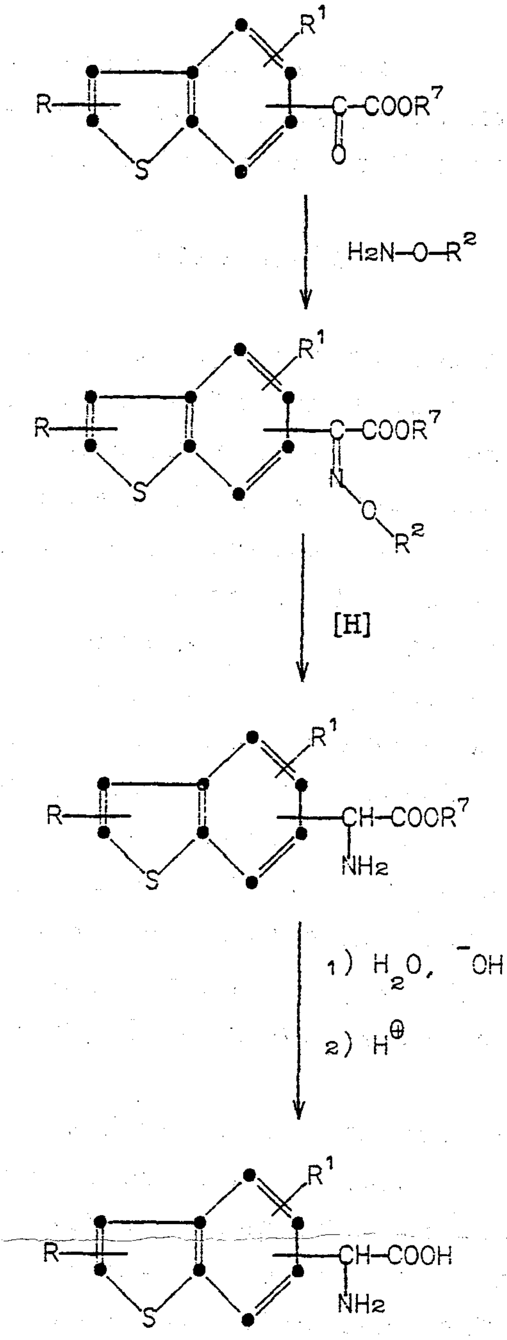


In the above formulae R, $R^1$, and $R^7$ have the same meanings as defined earlier and $R^2$ is hydrogen or $C_1$-$C_4$ alkyl.

Alternatively, the benzothienylglycine and dihydrobenzothienylglycine compounds are prepared with an acetic acid ester represented by the following:

$$R^4-CH_2COOR^7$$

The acetic acid ester is reacted in ethyl alcohol at a temperature of between about -50°C and about 0°C with n-butyl nitrite and sodium ethylate to form the oxime. The oxime is then reduced as described above and the ester is saponified to provide the benzothienylglycine or dihydrobenzothienylglycine. Prior to use in the acylation of a 7-amino nucleus compound, the amino group of the glycine derivative desirably is protected with a conventional amino-protecting group such as defined earlier. Likewise, the hydroxy group of the oxime desirably is protected in the acylation of a nucleus compound to form the compound of Formula IV. For example, the oxime hydroxy group may be protected with an acyl group e.g. chloroacetyl, or with an alkyl or substituted alkyloxycarbonyl group such as the t-BOC group.

The benzothienylglycyl and dihydrobenzothienylglycines compound may be in the D-, L- or D,L-form. Preferably, the ester or free acid is resolved or epimerized to the desired D-isomer and then used in the acylation. The resolution may be carried out, for example, with dibenzoyltartaric acid by the method of Lorenz, U.S. Patent No. 3,832,388 or by epimerization via the benzalimine by the method of Clark and Elks,

U.S. Patent No. 3,976,680. The separation of the D and L isomers also may be performed enzymatically by the procedure described in Methods in Enzymology, 44, 746 (1976). According to this method, the D,L-mixture as the N-chloroacetyl derivative is passed over a column packed with DEAE sephadex containing the enzyme N-acyl-L-aminoacid amidohydrolase. The L-N-chloroacetyl substrate is hydrolysed by the enzyme while the D-N-chloroacetyl derivative is not and is eluted as such with about 0.1M potassium hydrogen phosphate buffer (pH ca. 7.08). The desired D-N-chloroacetylglycine then is hydrolysed chemically to the 2-amino-2-(benzothienyl)-acetic acid or ester or the 2-amino-2-(2,3-dihydrobenzothienyl)acetic acid or ester.

The oximino cephalosporins, Formula (V), preferably have the oximino group as the syn isomer although the anti isomer also possesses significant antibacterial activity. The desired isomer is obtained with the oximino acid of the desired configuration. The syn and anti forms of the oximino acid may be obtained with the isomeric mixture by chromatography, especially $C_{18}$ HPLC.

In a further aspect of the invention, there is provided a cyclic derivative of the benzothienylglycyl (or 2,3-dihydrobenzothienyl) cephalosporins of Formula (I) represented by the following ($R^2$ and $R^3$ of Formula (I) are taken together to form

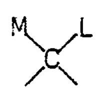 ) :

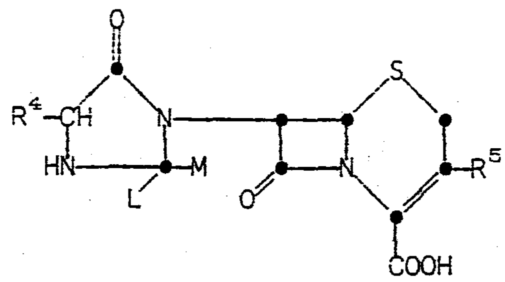

In the formula, $R^4$ and $R^5$ have the same meanings as defined earlier and L and M are, independently, $C_1$-$C_4$ alkyl. The cyclic derivatives are useful precursor compounds to the Formula (I) glycine derivatives because they are converted in vivo to the Formula (I) compounds upon absorption.

In the above formula, $C_1$-$C_4$ alkyl refers to methyl, ethyl, n-propyl, isopropyl, and n-butyl. These compounds, named generically herein as 7β-(2,2-dialkyl-5-oxo-4-benzothienyl- or 4-(2,3-dihydrobenzothienyl)-1-imidazolidinyl)-3H(or 3-substituted)-3-cephem-4-carboxylic acids, are prepared by the condensation of a dialkyl ketone with the compound of Formula (I) in the presence of a small amount of an acid such as p-toluenesulfonic acid or methanesulfonic acid. The rate of dehydration may be increased by heating the reaction mixture. The condensation is carried out in a suitable solvent which may be an excess of the ketone used to form the cyclic derivative. Ketones which may be used in the condensation are, for example, acetone, diethyl ketone, methylethyl ketone, or methyl-n-propyl ketone.

Preferred compounds of the formula above are those represented when L and M are methyl, for example, 7β-[2,2-dimethyl-5-oxo-4-(benzothien-4-yl)-1-imidazolidinyl]-3-methyl-3-cephem-4-carboxylic acid, and 7β-[2,2-dimethyl-5-oxo-4-(4-hydroxybenzothien-7-yl)-3-chloro-3-cephem-4-carboxylic acid.

This invention also provides pharmaceutical formulations which comprise as their active ingredient an antibiotic of Formula (I) or a pharmaceutically-acceptable salt thereof. According to this aspect of the invention, the antibiotic compound of Formula (I), or a pharmaceutically-acceptable salt thereof, may be formulated into suitable dosage forms such as capsules, pulvules, tablets or liquid suspensions suitable for oral administration. The composition may also contain an excipient, antioxidant, stabilizer, lubricant, suspending agent and the like. Such formulations may contain starch, or a sugar such as sucrose, glucose or mannose. Suspensions may contain a flavoring agent, buffer, suspending agent and the like. Suitable dosage forms may comprise gelatin capsules containing 250 mg. or 500 mg. of the antibiotic.

The oximino compounds may also be prepared in suitable dosage form for parenteral administration. For example, the compound may be formulated in hermetically-sealed vials or in sterile rubber-stoppered ampoules for i.m. or i.v. administration. Likewise, the oximino compounds may be formulated in plastic bags (pouches) for i.v. administration by the drip method. For such usage the antibiotic is formulated or mixed with a

physiologically-acceptable fluid such as Water-for-Injection, 5% dextrose, or 0.9% saline. Suitable parenteral dosage forms of the oximino cephalosporins may comprise 250 mg of the antibiotic as the sodium salt in a glass ampoule. Another formulation comprises 500 mg of the potassium salt in a glass ampoule.

The following non-limiting examples are provided to further illustrate the invention.

## Example 1

7β-[D-2-Amino-2-(benzothien-4-yl)acetylamino]-3-methyl-3-cephem-4-carboxylic acid

A suspension of 25 mg of α-t-butyloxycarbonylamino-α-(benzothien-4-yl)acetic acid and 18 mg of EEDQ in 3 ml of acetonitrile was sonicated for 3 minutes and then added to a solution of 26 mg of p-nitrobenzyl 7-amino-3-methyl-3-cephem-4-carboxylate in 30 ml of acetonitrile maintained at 0°C. The acylation mixture was stirred for about 5 hours. Thin layer chromatography on an aliquot of the mixture showed that the acylation was complete. The reaction mixture was added to the reaction mixture of a larger scale acylation carried out with 770 mg of 7-ADCA pNB ester, 670 mg of the α-t-BOC amino-(benzothien-4-yl)-acetic acid and 500 mg EEDQ in acetonitrile in substantially the manner described above. The combined reaction mixtures were evaporated to dryness to an oil. The oil was dissolved in ethyl acetate and the solution washed with a saturated solution of sodium bicarbonate and then

with 1N hydrochloric acid. The solution was dried over magnesium sulfate and evaporated to dryness to yield 1.3 g (87% yield) of p-nitrobenzyl D,L-7β-[2-(t-butyloxycarbonylamino)-(benzothien-4-yl)-acetylamino]-3-methyl-3-cephem-4-carboxylate.

The amino-protected and esterified acylation product (1.3 g in THF) was hydrogenated for one hour at room temperature over 2.0 g of prereduced 5% Pd/C under 55 psi hydrogen pressure. After the deesterification was complete, the reduction mixture was filtered and evaporated to an oil. The oil was dissolved in a mixture of ethyl acetate and pH 7 buffer. The pH was adjusted to 8 and the aqueous layer separated. The organic layer was extracted with aqueous sodium bicarbonate and the extract was combined with the aqueous (pH 8) layer. The pH of the combined aqueous layers were acidified to pH 2.3 and extracted with ethyl acetate. The extract was dried over magnesium sulfate and evaporated to dryness to yield 0.9 g of D,L-7β-[2-(t-butyloxycarbonylamino)-(benzothien-4-yl)acetylamino]-3-methyl-3-cephem-4-carboxylic acid.

The amino-protected deesterified product, 0.9 g, was treated with 5 ml of trifluoroacetic acid to remove the t-BOC group. The acid solution was diluted with water and the pH of the solution was adjusted to 6.0 with ammonium hydroxide. The solution was then lyophilized to the title compound as a crude solid.

The solid product was dissolved in acetonitrile:water (9:1, v:v) and the pH of the solution was adjusted to 6.0. The product crystallized from the solution as white crystals which were collected by filtration and dried overnight under vacuum.

NMR ($d_1$-trifluoroacetic acid): δ 2.33 (s, 3H), 3.34 (d, J = 8Hz, 2H), 3.46 (d, J = 8Hz, 2H), 4.12 (s, 1H), 5.20 (d, J = 4Hz, 1H), 5.80 (d, J = 4Hz, 1H), and 7.5-8.2 (m, 5H).

The product was evaluated by the agar dilution method for antibacterial activity against representative strains of gram positive bacteria. Table I below lists the minimum inhibitory concentrations (MIC) in micrograms per milliliter of both the title compound and cephalexin against staphylococci, streptococci and H. influenzae strains.

TABLE 1

Antibacterial Activity

Title Compound[1]

| Test Microorganism | Strain | Compound A[2] | Compound B[3] |
|---|---|---|---|
| Staphlococcus aureus | X1.1 | 2 | 4 |
| Staphlococcus aureus | V41 | 16 | 128 |
| Staphlococcus aureus | X400 | 64 | 128+ |
| Staphlococcus aureus | S13E | 16 | 128 |
| Staphlococcus epidermidis | EPI1 | 16 | 32 |
| Staphlococcus epidermidis | 222 | 8 | 8 |
| Streptococcus pyogenes | C203 | .25 | .5 |
| Streptococcus pneumoniae | Park I | 1 | 2 |
| Streptococcus group D | X66 | 64 | 128 |
| Streptococcus group D | 2041 | 32 | 128 |
| Haemophilus influenzae | C.L. | 4 | 8 |
| Haemophilus influenzae | 76 | 2 | 8 |

[1]Numerical values are MIC in mcg./ml.

[2]A = 7β-[D-2-Amino-2-(benzothien-4-yl)acetylamino]-3-methyl-3-cephem-4-carboxylic acid (from Example 1).

[3]B = Cephalexin = 7β-(D-2-amino-2-phenylacetylamino)-3-methyl-3-cephem-4-carboxylic acid.

## Example 2

7β-[2-Amino-2-(3-methylbenzothien-7-yl)-acetylamino]-3-methyl-3-cephem-4-carboxylic acid

To a solution of 1.050 g of p-nitrobenzyl 7-aminodesacetoxycephalosporanate in 300 ml of dry acetonitrile cooled to 0°C. and maintained under nitrogen was added a solution of 921 mg of α-t-butyloxycarbonylamino-α-(3-methylbenzothien-7-yl)acetic acid in 75 ml of dry acetonitrile containing 741 mg of EEDQ. The reaction mixture was stirred overnight under a drying tube and then evaporated to dryness. The residual oil was dissolved in ethyl acetate and the solution washed with 1N hydrochloric acid and a saturated solution of sodium bicarbonate, dried, and evaporated to dryness to provide 1.62 g of crude product as an oil.

The product was deesterified with prereduced 5% palladium on carbon catalyst as follows. The ester, 1.62 g, was dissolved in 75 ml of THF and a suspension of 1.62 g. of 5% Pd/C in 40 ml of methyl alcohol (prereduced under 54 psi $H_2$ for 30 minutes) was added to the solution. The reduction was carried out at room temperature for 45 minutes under 56 psi hydrogen pressure. The reduction mixture was filtered and the filtrate evaporated to dryness. The crude product was dissolved in ethyl acetate-water and the pH of the mixture adjusted to 7.8. The aqueous layer was separated, washed with ethyl acetate and acidified to pH 2.2. The

deesterified product was extracted from the aqueous layer with ethyl acetate and the extract dried, and evaporated to yield 0.72 g of the amino-protected (t-BOC) free acid.

The product was deblocked by treatment with 8 ml of trifluoroacetic acid for 5 minutes. The reaction mixture was diluted with 100 ml of water and the pH adjusted to 7.0 with dilute ammonium hydroxide. The solution was then filtered and chromatographed on a Waters Associates reverse phase $C_{18}$ silica HPLC using as a gradient, 0-20% acetonitrile-1% acetic acid-water. Multiple fractions were collected and lyophilized. Fractions 33-37 were combined and contained the L isomer (side chain asymmetric center) and fractions 29-31 contained the D isomer.

## Example 3

7β-[2-Amino-2-(benzothien-5-yl)acetylamino]-3-methyl-3-cephem-4-carboxylic acid

To a solution of 100 mg of p-nitrobenzyl 7-aminodesacetoxycephalosporanate in 3 ml of dry acetonitrile cooled to 0°C and maintained under a nitrogen atmosphere was added a solution of α-t-butyloxycarbonylamino-α-(benzothien-5-yl)acetic acid in 6 ml of THF containing 70 mg of EEDQ. The reaction was stirred overnight and then evaporated to dryness. The residue was dissolved in ethyl acetate, washed with 1N hydrochloric acid, saturated sodium bicarbonate solution, then dried and evaporated to dryness to yield 158 mg

(95%) of crude product. This product was combined with 170 mg of the same product obtained in a similar manner. The combined products (300 mg), when chromatographed over silica gel eluted with 15% ethyl acetate:toluene, produced 100 mg of the D-(t-BOC)-protected product as the p-nitrobenzyl ester. Also obtained was 107 mg of the L-form and 25 mg of the D,L- mixture.

D-form:

NMR (δ, 90MHz, $CDCl_3$) 1.4 (s, 9H, t-Boc), 2.1 (s, 3H, $C_3$-$H_3$), 3.24 (ABq, 2H, $C_2$-$H_2$), 4.90 (d, 1H, $C_6$-H), 5.28 (s, 2H, $pNBCH_2$), 5.38 (s, 1H, α-CH), 5.74 (m, 3H, $C_7$-H and amide NH), 6.82 (d, 1H, amide NH), 7.12-8.24 (m, 9H, aromH)

L-form:

NMR (δ, 90MHz, $CDCl_3$) 1.4 (s, 9H, t-Boc), 2.1 (s, 3H, $C_3$-$H_3$), 3.24 (ABq, 2H, $C_2$-$H_2$), 4.88 (d, 1H, $C_6$-H), 5.22 (s, 2H, $pNHCH_2$), 5.34 (s, 1H, α-CH, 5.62 (m, 3H, $C_7$-H and amide NH), 6.84 (d, 1H, amide NH), 7.08-8.20 (m, 9H, aromH)

After removal of the t-butoxycarbonyl group from the product above the p-nitrobenzyl ester was removed as follows.

The palladium on carbon (5%) catalyst (62 mg) was pre-reduced in 10 ml of ethanol for 30 minutes at 60 psi of hydrogen. To this suspension was added 61.3 mg of the p-nitrobenzyl 7-β-[2-amino-2-(benzothien-5-yl)-acetylamino]-3-methyl-3-cephem-4-carboxylate from above, dissolved in 40 ml of methanol and .2 ml of 1N HCl. The hydrogenation was allowed to continue for 1.5 hours at

60 psi of hydrogen, after which .2 ml of hydrochloric acid was added. The mixture was stirred for an additional 5 minutes after which the catalyst was removed by filtering. The catalyst was washed with 4 ml of 1:1 methanol/$H_2O$ and then methanol alone. The organic layers were combined and dissolved in ethyl acetate. The pH was adjusted to pH 7 with 1N NaOH, the organic layer was removed and the aqueous layer was again extracted with ethyl acetate. The aqueous layer was then acidified to pH 4.25 with 1N HCl and then lyophilized. The residue was suspended in about 2 ml of $H_2O$ and the pH adjusted to 4.25 after which the suspension was filtered to yield 30 mg (65%) of the final product.

D-form:

NMR (δ, 270MHz, $D_2O$/DCl) 1.97 (s, 3H, $C_3$-$H_3$), 3.16 (ABq, 2H, $C_2$-$H_2$), 5.05 (d, 1H, $C_6$H), 5.4 (s, 1H, α-CH, 5.58 (m, 1H, $C_7$-H), 7.35-8.05 (m, 5H, aromH)

CLAIMS

1. A compound of Formula (I):

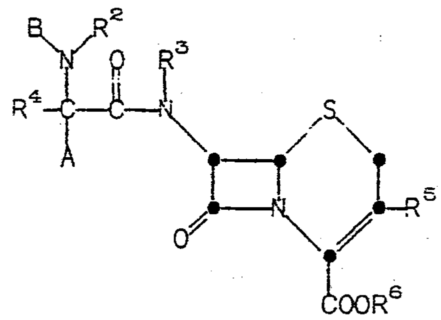

(I)

in which $R^4$ is

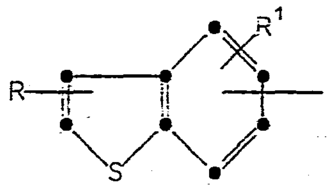

or

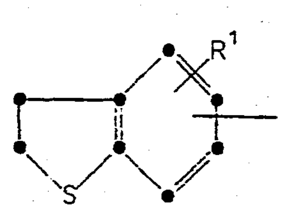

R is hydrogen, $C_1$-$C_3$ alkyl, halogen, nitro, amino, $C_1$-$C_4$ alkoxy, $C_1$-$C_4$ alkanoylamino, or $C_1$-$C_3$ alkylsulfonylamino;

$R^1$ is hydrogen, $C_1$-$C_3$ alkyl, halogen, hydroxy, or $C_1$-$C_4$ alkoxy;

A and B both are hydrogen, or taken together complete a double bond;

$R^2$ is hydrogen, an amino-protecting group, hydroxy, $C_1$-$C_4$ alkoxy or a carboxy-substituted alkoxy group or carboxy-substituted cycloalkoxy group of the formula

$$-\overset{c}{\underset{d}{C}}-(CH_2)_n-COOR^6$$

in which c and d, independently, are hydrogen or $C_1$-$C_3$ alkyl, or c and d taken together with the carbon to which they are bonded form a $C_3$-$C_6$ cycloalkyl ring; and n is 0 to 3;

$R^3$ is hydrogen, or $R^2$ and $R^3$ taken together are

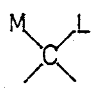

in which M and L, independently, are $C_1$-$C_4$ alkyl; provided that $R^2$ is hydroxy, $C_1$-$C_4$ alkoxy, carboxy-substituted alkoxy or carboxy-substituted cycloalkoxy only when A and B complete a double bond, and that A and B, both are hydrogen when $R^3$ is other than hydrogen;

$R^5$ is hydrogen, methyl, halogen, methoxy, vinyl, or a substituted methyl group represented by the formula

$$-CH_2-R^{5'}$$

in which $R^{5'}$ is methoxy, acetoxy, or carbamoyloxy;

$R^6$ is hydrogen, or a carboxy-protecting group; or a pharmaceutically-acceptable salt thereof.

2. A compound of Formula (I), as claimed in claim 1, in which $R^2$ and $R^3$, when taken together, form

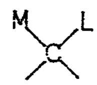

;

or a pharmaceutically-acceptable salt thereof.

3. A compound of Formula (I), as claimed in claim 1, in which A and B, when taken together, complete a double bond, and $R^2$ is methoxy; or a pharmaceutically-acceptable salt thereof.

4. A compound of Formula (I), as claimed in any one of claims 1 to 3, in which $R^4$ is

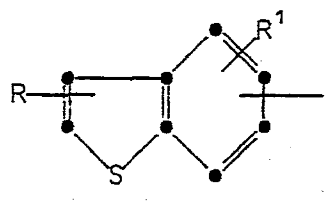

;

or a pharmaceutically-acceptable salt thereof.

5. A compound of Formula (I), as claimed in any one of claims 1 to 3 in which $R^4$ is

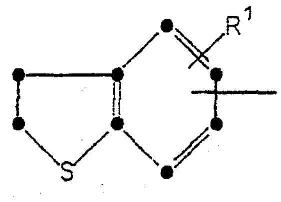

;

or a pharmaceutically-acceptable salt thereof.

6. A compound of Formula (I), as claimed in claims 4 or 5, in which $R^1$, is hydrogen, halogen, hydroxy, $C_1$-$C_4$ alkoxy; or a pharmaceutically-acceptable salt thereof.

7. A compound of Formula (I), as claimed in claim 6 in which R or $R^1$ is, independently hydrogen or halo; or a pharmaceutically-acceptable salt thereof.

8. A compound of Formula (I), as claimed in claim 6 or 7, in which R and $R^1$ both are hydrogen; or a pharmaceutically-acceptable salt thereof.

9. A compound of Formula (I), as claimed in any one of claims 1 to 8, in which $R^5$ is methyl, chloro, methoxy, hydrogen, methoxymethyl, or vinyl; or a pharmaceutically-acceptable salt thereof.

10. A compound of Formula (I), as claimed in claim 9, in which $R^5$ is methyl or chloro; or a pharmaceutically-acceptable salt thereof.

11. A compound of Formula (I), as claimed in any one of claims 1 to 10, in which $R^6$ is hydrogen; or a pharmacuetically-acceptable salt thereof.

12. 7β-[D-2-Amino-2-(benzothien-4-yl)acetylamino]-3-methyl-3-cephem-4-carboxylic acid; or a pharmaceutically-acceptable salt thereof.

13. 7β-[2-Amino-2-(3-methylbenzothien-7-yl)-acetylamino]-3-methyl-3-cephem-4-carboxylic acid, or a pharmaceutically-acceptable salt thereof.

14. 7β-[2-Amino-2-(benzothien-5-yl)acetylamino]-3-methyl-3-cephem-4-carboxylic acid; or a pharmaceutically-acceptable salt thereof.

15. A process for preparing a compound of Formula (I), as claimed in any one of claims 1 to 14, which comprises

(A) acylating a compound of Formula (II):

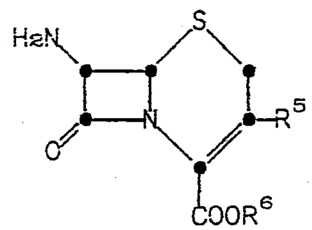

(II)

with an acylating agent of Formula (III):

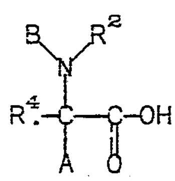

(III),

or an activated derivative thereof, in which A, B, $R^2$, $R^4$, $R^5$ and $R^6$ are as defined in claim 1, followed, optionally, by removal of any amino- or carboxyl-protecting groups present;

(B) deblocking of a protected acid of Formula (I) in which $R^6$ is a carboxy-protecting group to provide a compound of Formula (I) in which $R^6$ is hydrogen;

(C) removal of an amino-protecting group, $R^2$, from a compound of Formula (I) to provide a compound of Formula (I) in which $R^2$ is hydrogen;

(D) when it is desired to form a compound in which $R^2$ and $R^3$, when taken together form a group of the Formula:

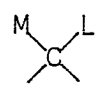,

reacting a compound of Formula (I), in which $R^2$ and $R^3$ both are hydrogen, with a ketone of the Formula:

M-C(O)-L

in which M and L are as defined in claim 1 or,

(E) reducing a compound of Formula (I) in which A and B are taken together to form a double bond and $R^2$ is hydroxy or methoxy, to produce a compound of Formula (I) in which A, B, and $R^2$ are hydrogen; and,

(F) if desired, salifying a compound of formula (I),

(G) or, if desired, converting a salt of a compound of Formula (I) to the free amine or acid.

16. A compound of Formula (I), or a pharmaceutically-acceptable salt thereof, as claimed in any one of claims 1 to 14, for use as an antibiotic in the chemotherapy of warm-blooded animals.

17. A pharmaceutical formulation which comprises as an active ingredient, a compound of Formula (I), or a pharmaceutically-acceptable salt thereof, as claimed in any one of claims 1 to 14 associated with one or more pharmaceutically-acceptable carriers or vehicles therefor.

18. A tablet, capsule or oral suspension containing as its active ingredient, a compound of Formula (I), or a pharmaceutically-acceptable salt thereof, as claimed in any one of claims 1 to 14.

## CLAIMS

1. A process for preparing a compound of Formula (I):

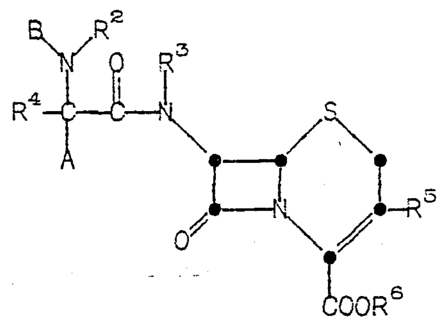

(I)

in which $R^4$ is

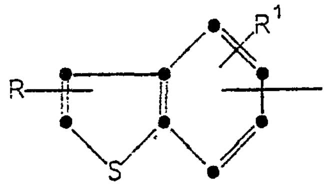

or

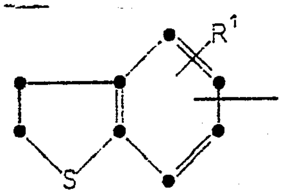

R is hydrogen, $C_1$-$C_3$ alkyl, halogen, nitro, amino, $C_1$-$C_4$ alkoxy, $C_1$-$C_4$ alkanoylamino, or $C_1$-$C_3$ alkylsulfonylamino;

$R^1$ is hydrogen, $C_1$-$C_3$ alkyl, halogen, hydroxy, or $C_1$-$C_4$ alkoxy;

A and B both are hydrogen, or taken together complete a double bond;

$R^2$ is hydrogen, an amino-protecting group, hydroxy, $C_1$-$C_4$ alkoxy or a carboxy-substituted alkoxy group or carboxy-substituted cycloalkoxy group of the formula

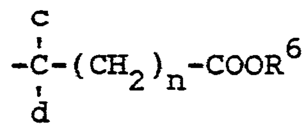

in which c and d, independently, are hydrogen or $C_1$-$C_3$ alkyl, or c and d taken together with the carbon to which they are bonded form a $C_3$-$C_6$ cycloalkyl ring; and n is 0 to 3;

$R^3$ is hydrogen, or $R^2$ and $R^3$ taken together are

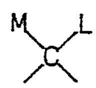

in which M and L, independently, are $C_1$-$C_4$ alkyl; provided that $R^2$ is hydroxy, $C_1$-$C_4$ alkoxy, carboxy-substituted alkoxy or carboxy-substituted cycloalkoxy only when A and B complete a double bond, and that A and B, both are hydrogen when $R^3$ is other than hydrogen;

$R^5$ is hydrogen, methyl, halogen, methoxy, vinyl, or a substituted methyl group represented by the formula

$$-CH_2-R^{5'}$$

in which $R^{5'}$ is methoxy, acetoxy, or carbamoyloxy;

$R^6$ is hydrogen, or a carboxy-protecting group; or a pharmaceutically-acceptable salt thereof, which comprises

(A) acylating a compound of Formula (II):

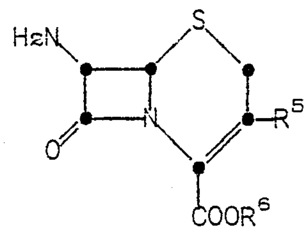

(II)

with an acylating agent of Formula (III):

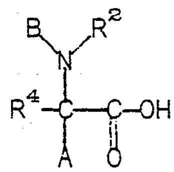

(III),

or an activated derivative thereof, in which A, B, $R^2$, $R^4$, $R^5$ and $R^6$ are as defined above, followed, optionally, by removal of any amino- or carboxyl-protecting groups present;

(B) deblocking of a protected acid of Formula (I) in which $R^6$ is a carboxy-protecting group to provide a compound of Formula (I) in which $R^6$ is hydrogen;

(C) removal of an amino-protecting group, $R^2$, from a compound of Formula (I) to provide a compound of Formula (I) in which $R^2$ is hydrogen;

(D) when it is desired to form a compound in which $R^2$ and $R^3$, when taken together form a group of the Formula:

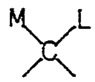 ,

reacting a compound of Formula (I), in which $R^2$ and $R^3$ both are hydrogen, with a ketone of the Formula:

M-C(O)-L

in which M and L are as defined above or,

(E) reducing a compound of Formula (I) in which A and B are taken together to form a double bond and $R^2$ is hydroxy or methoxy, to produce a compound of Formula (I) in which A, B, and $R^2$ are hydrogen; and,

(F) if desired, salifying a compound of formula (I),

(G) or, if desired, converting a salt of a compound of Formula (I) to the free amine or acid.

2. A process for preparing a compound of Formula (I), as claimed in claim 1, in which $R^2$ and $R^3$, when taken together, form

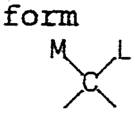 ;

or a pharmaceutically-acceptable salt thereof.

3. A process for preparing a compound of Formula (I), as claimed in claim 1, in which A and B, when taken together, complete a double bond, and $R^2$ is methoxy; or a pharmaceutically-acceptable salt thereof.

4. A process for preparing a compound of Formula (I), as claimed in any one of claims 1 to 3, in which $R^4$ is

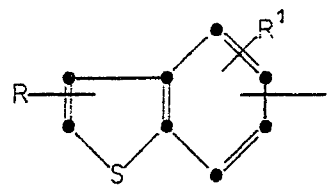

;

or a pharmaceutically-acceptable salt thereof.

5. A process for preparing a compound of Formula (I), as claimed in any one of claims 1 to 3 in which $R^4$ is

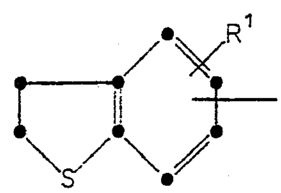

;

or a pharmaceutically-acceptable salt thereof.

6. A process for preparing a compound of Formula (I), as claimed in claims 4 or 5, in which $R^1$, is hydrogen, halogen, hydroxy, $C_1$-$C_4$ alkoxy; or a pharmaceutically-acceptable salt thereof.

7. A process for preparing a compound of Formula (I), as claimed in claim 6 in which R or $R^1$ is, independently hydrogen or halo; or a pharmaceutically-acceptable salt thereof.

8. A process for preparing a compound of Formula (I), as claimed in claim 6 or 7, in which R and $R^1$ both are hydrogen; or a pharmaceutically-acceptable salt thereof.

9. A process for preparing a compound of Formula (I), as claimed in any one of claims 1 to 8, in which $R^5$ is methyl, chloro, methoxy, hydrogen, methoxymethyl, or vinyl; or a pharmaceutically-acceptable salt thereof.

10. A process for preparing a compound of Formula (I), as claimed in claim 9, in which $R^5$ is methyl or chloro; or a pharmaceutically-acceptable salt thereof.

11. A process for preparing a compound of Formula (I), as claimed in any one of claims 1 to 10, in which $R^6$ is hydrogen; or a pharmacuetically-acceptable salt thereof.

12. A process as claimed in claim 1 for preparing 7β-[D-2-Amino-2-(benzothien-4-yl)acetylamino]-3-methyl-3-cephem-4-carboxylic acid; or a pharmaceutically-acceptable salt thereof.

13. A process as claimed in claim 1 for preparing 7β-[2-Amino-2-(3-methylbenzothien-7-yl)acetylamino]-3-methyl-3-cephem-4-carboxylic acid, or a pharmaceutically-acceptable salt thereof.

14. A process as claimed in claim 1 for preparing 7β-[2-Amino-2-(benzothien-5-yl)acetylamino]-3-methyl-3-cephem-4-carboxylic acid; or a pharmaceutically-acceptable salt thereof.

15. A compound of Formula (I), or a pharmaceutically-acceptable salt thereof, whenever prepared by a process as claimed in any one of claims 1 to 14.